Europäisches Patentamt

European Patent Office

Office européen des brevets

(19)

(11) Numéro de publication : **0 190 516**
**B1**

(12)

# FASCICULE DE BREVET EUROPÉEN

(45) Date de publication du fascicule du brevet :
15.06.88

(51) Int. Cl.⁴ : **B 01 J   8/02**, C 01 C   1/04//
C07C29/15

(21) Numéro de dépôt : 85402334.8

(22) Date de dépôt : 28.11.85

(54) Réacteur et procédé de synthèse catalytique exothermique, en phase gazeuse et sous pression.

(30) Priorité : 03.12.84 FR 8418378

(43) Date de publication de la demande :
13.08.86 Bulletin 86/33

(45) Mention de la délivrance du brevet :
15.06.88 Bulletin 88/24

(84) Etats contractants désignés :
DE FR GB IT NL

(56) Documents cités :
DE-C-   911 490
FR-A- 2 183 985
US-A- 4 452 760

(73) Titulaire : SOCIETE CHIMIQUE DE LA GRANDE
PAROISSE, AZOTE ET PRODUITS CHIMIQUES
8, rue Cognacq-Jay
F-75007 Paris (FR)

(72) Inventeur : Lesur, Pierre
20, avenue Rapp
F-75007 Paris (FR)
Inventeur : Faury, Christian
16, avenue Victor Hugo
F-92170 Vanves (FR)
Inventeur : Lafleur, Guy
2, rue du Bordeau Saint-Rémy-l'Honoré
F-78690 Les Essarts-le-Roi (FR)
Inventeur : Papillon, André
32, avenue Henri Martin
F-94400 Saint-Maur-des-Fossés (FR)

(74) Mandataire : Bouton Neuvy, Liliane et al
L'Air liquide, Société Anonyme pour L'Etude et
L'Exploitation des Procédés Georges Claude 75,
Quai d'Orsay
F-75321 Paris Cédex 07 (FR)

EP 0 190 516 B1

Jouve, 18, rue St-Denis, 75001 Paris, France

## Description

La présente invention concerne la synthèse catalytique exothermique en phase gazeuse sous pression et plus particulièrement un type de réacteur horizontal permettant de réaliser avantageusement des synthèses, notamment la synthèse d'ammoniac à partir d'azote et d'hydrogène, et celle du méthanol.

Depuis plusieurs décades on conduit les réactions catalytiques exothermiques d'équilibre en phase gazeuse dans des réacteurs de synthèse contenant traditionnellement plusieurs couches de catalyseur entre lesquelles ou au sein desquelles sont disposés des moyens de contrôle et de régulation des températures de réaction. Le refroidissement soit continu au sein de la masse catalytique, soit discontinu entre couches permet d'éloigner les conditions réactionnelles de celles de l'équilibre et donc d'augmenter la vitesse de réaction.

Les contraintes imposées par les conditions économiques ont orienté les recherches vers des installations ayant pour finalité la diminution de la consommation énergétique de la boucle de synthèse, ou plus généralement de la consommation globale de l'atelier de fabrication de l'ammoniac ou du méthanol.

On a notamment proposé la diminution de la pression des synthèses exothermiques en phase gazeuse, ce qui entraîne la construction d'ateliers de taille de plus en plus importante.

L'augmentation du taux de conversion a également été suggérée. Ce taux peut être accru en remplaçant le contrôle des températures par trempe entre lits catalytiques par un ou plusieurs échangeurs internes. La conception mécanique compliquée de tels réacteurs engendre un surcoût élevé et une fiabilité incertaine, et les opérations de chargement et de vidange du catalyseur en sont rendues plus difficiles. Les réacteurs multicouches les plus courants ne se prêtent pas facilement à l'introduction d'un ou plusieurs échangeurs intercouches en plus de l'échangeur de sortie (complexité, risque de fuites internes).

Le taux de conversion a aussi été accru en diminuant la granulométrie du catalyseur, ce qui augmente son activité mais aussi sa perte de charge.

Le taux de conversion a été également augmenté par accroissement du volume du réacteur. Les réacteurs verticaux à circulation axiale ne permettent que de façon limitée cette évolution, leur perte de charge devenant rédhibitoire au-delà de 70 m³ environ de catalyseur. Les réacteurs verticaux à circulation radiale ainsi que les réacteurs horizontaux multicouches à circulation verticale permettent une augmentation du volume du catalyseur en allongeant le récipient sans augmenter le diamètre. Les réacteurs multicouches radiaux ainsi que de nombreux réacteurs axiaux présentent des inconvénients de fiabilité et de risque de fuites dus à une ouverture égale au diamètre de la virole de force, en vue de l'introduction et du retrait de l'équipement interne lors du changement de catalyseur. L'étanchéité des brides de grand diamètre est difficile à réaliser. Le diamètre est en général limité par celui de l'ouverture à cause de problèmes de construction et des gabarits admissibles pour le transport. De plus, l'augmentation du volume du catalyseur des réacteurs radiaux et horizontaux multicouches conduit à des équipements internes très longs posant des problèmes de construction, de fiabilité et de renouvellement de catalyseur.

Un autre inconvénient des réacteurs multicouches réside dans le fait que leur taux de remplissage : rapport du volume de catalyseur au volume de l'enceinte sous haute pression, diminué du volume des échangeurs internes, n'est environ que de 0,5.

L'évolution des technologies et des conditions économiques dans le secteur de la construction mécanique tend à rendre les équipements internes complexes en acier inoxydables très coûteux et au contraire la technique simple des récipients dits unicouches, en série, compétitive. Cette dernière met en œuvre non pas un grand réacteur, mais des réacteurs verticaux de petite ou moyenne dimension, contenant une seule couche de catalyseur, en série, et reliés entre eux par des tuyauteries, les échangeurs étant enfermés dans des récipients indépendants. Cette technique ne présente pas tous les inconvénients des réacteurs multicouches, mais elle ne se prête pas aisément à l'emploi d'importants volumes de catalyseur, la perte de charge augmentant vite en raison de la circulation axiale des gaz. Par contre, les réacteurs unicouches horizontaux à circulation verticale se prêtent beaucoup mieux à l'utilisation de grands volumes catalytiques.

Dès 1944, à une époque où la production d'ammoniac était mise en œuvre dans des capacités catalytiques de faible volume, le brevet allemand 911.490, avait déjà proposé un réacteur horizontal contenant une seule couche de catalyseur dans lequel les gaz sous haute pression et température de la réaction sont en contact avec la virole de force. Ces conditions de fonctionnement nécessitent l'emploi d'aciers fortement alliés très difficiles à mettre en œuvre dans de gros diamètres. Ce type de réacteur n'est pas transposable dans des grandes unités de synthèse.

Plus récemment, le brevet français 2.183.985 a décrit un réacteur horizontal pour la synthèse de l'ammoniac, avec un échangeur thermique vertical interne-externe et deux chambres réactives avec lits de catalyseur. Cet échangeur vertical assure un échange thermique entre les courants gazeux. Cette solution apparaît comme manifestement compliquée du point de vue équipement interne, elle présente les inconvénients inhérents aux réacteurs multicouches d'un coût de réalisation élevé, d'une fiabilité relative, et d'une manutention assez difficile du catalyseur avec un taux de remplissage limité.

On a cherché à améliorer la technique des récipients unicouches en s'affranchissant de ses limites et contraintes, en alliant un taux de conversion très élevé à une perte de charge faible dans le catalyseur, ceci quel que soit le volume de catalyseur, tout en recourant à des techniques de construction très simples et éprouvées ; ces avantages étant valables quelle que soit la taille de l'installation. Le type de réacteur proposé est compatible avec l'abaissement de pression de synthèse et l'augmentation de volume de catalyseur consécutive, jusque vers 100 à 150 m³ par exemple. La conception de l'équipement interne, les technologies de construction, montage, manipulation du catalyseur, exploitation sans fuites de gaz sont d'une grande simplicité.

L'invention consiste en la conception de réacteurs de synthèse, unicouches, cylindriques, horizontaux, dépourvus d'échangeurs internes dont les parois sous haute pression ne sont pas portées à des températures très élevées par les gaz à leur contact. Ainsi, les parois ne travaillent pas au fluage. Ce résultat, obtenu sans compliquer le circuit de circulation des gaz dans l'ensemble du réacteur, permet de diminuer la température de calcul de la virole de force de 550 °C jusqu'à 450 °C, voire 300 °C et inférieure suivant les cas. Au démarrage de l'installation, il est généralement nécessaire d'introduire dans la chambre de catalyse, au moment du démarrage, du gaz de synthèse à une température pouvant approcher 500 °C et la température de l'effluent gazeux du lit catalytique peut excéder légèrement les 500 °C. La présente technique évite le contact de ces deux flux gazeux très chauds avec la virole sous pression.

L'objet principal de l'invention concerne un appareil et un procédé de synthèse en catalyse hétérogène exothermique en phase gazeuse sous pression mis en œuvre dans ce type d'appareillage.

Le réacteur cylindrique horizontal à température de paroi contrôlée est constitué principalement par une virole principale sous haute pression, ou corps de force composée d'une partie cylindrique et de deux fonds hémisphériques contenant une enceinte porte-catalyseur concentrique dont la paroi externe est revêtue d'un isolant, un espace étant ménagé entre la paroi interne du corps de force et celle de la paroi externe de l'enceinte porte-catalyseur revêtue d'isolant, reposant sur un enroulement métallique hélicoïdal, d'une extrémité à l'autre de la virole, ledit enroulement s'appuyant lui-même sur la paroi interne de la virole sous haute pression. La virole principale sous haute pression et l'enceinte porte-catalyseur sont munies de moyens d'introduction et d'évacuation des flux gazeux, aux extrémités du grand axe et disposées en vis-à-vis sur les fonds hémisphériques, en outre, l'enceinte porte-catalyseur étant équipée d'une tubulure verticale de remplissage et vidange du catalyseur.

Suivant les cas ce type de réacteur permet d'utiliser une température de calcul de la virole de force inférieure ou égale à 300 °C et au plus de l'ordre de 450 °C, en raison de la protection thermique de la paroi de la virole principale sous haute pression.

La paroi de la virole principale sous haute pression est protégée thermiquement de deux manières, avec d'une part le revêtement extérieur isolant de l'enceinte interne porte-catalyseur, et d'autre part, par un courant de gaz isotherme de température moins élevée que celle du gaz contenu dans l'enceinte interne porte-catalyseur, ledit gaz isotherme circulant de façon hélicoïdale entre la paroi de force et celle revêtue de son isolant. Ce gaz n'est autre que le gaz réactif d'alimentation sous pression du réacteur.

Le revêtement extérieur isolant de l'enceinte porte-catalyseur ayant une haute résistance à la compression dans les conditions de service, assume la fonction de supportage de l'enceinte, en appui sur l'enroulement hélicoïdal, ce qui permet de supprimer les ponts thermiques entre celle-ci et la virole résistant à la pression. L'isolant est lui-même enfermé dans des viroles de protection mécanique, concentriques à l'enceinte porte-catalyseur, s'appuyant sur des frettes-supports circulaires non représentées, dans lesquelles sont ménagés des orifices assurant le passage du gaz de protection de la virole de force à circulation hélicoïdal. En outre, cet isolant est avantageusement inerte et insensible à l'eau introduite lors de la vidange du catalyseur.

Pour maintenir la paroi interne de la virole principale à une température égale ou inférieure à 300 °C, on fait passer un courant d'alimentation vers 200 °C, 120-250 °C, dans l'espace ménagé entre la virole sous pression et l'enceinte interne contenant le catalyseur, suivant une trajectoire hélicoïdale, d'une extrémité à l'autre du récipient. Ce gaz ressort ensuite du réacteur, puis est réchauffé avant son introduction dans l'enceinte catalytique.

Ce matelas de gaz à 200 °C est quasiment isotherme en raison du revêtement isolant de l'enceinte porte-catalyseur. Et, la température de calcul de 300 °C autorise l'emploi d'aciers faiblement alliés, de mise en œuvre facile, travaillant très en dessous de leur limite élastique, d'épaisseur modérée.

Selon une variante de la technique, et dans le but de simplifier au maximum l'équipement interne de l'appareillage, le courant de gaz de protection est introduit dans l'espace ménagé entre la virole sous pression et l'enceinte interne après avoir été réchauffé dans un échangeur extérieur jusqu'à la température d'entrée en réaction, par exemple 400 °C, dans le cas de la synthèse d'ammoniac. Le circuit du gaz de protection isotherme est le même que précédemment, excepté que le gaz ne ressort pas du réacteur, il est introduit directement dans l'enceinte porte-catalyseur entre 370 et 420 °C. Cette enceinte interne étant toujours revêtue extérieurement d'un isolant, de sorte que le matelas gazeux soit maintenu à environ 400 °C ; la température de calcul de la virole sous pression peut donc être de 450 °C, alors que le gaz traversant l'enceinte catalytique peut atteindre 500 °C.

Les aciers de construction sont moins alliés que si la température de calcul était de 550 °C, comme dans d'autres types de réacteur, et ils travaillent encore en-dessous de leur limite élastique, l'épaisseur de la paroi sous pression reste ainsi raisonnable et la mise en œuvre aisée.

Dans ces deux types de fonctionnement, les aciers sont soumis à des conditions éloignées de la corrosion à chaud par l'hydrogène.

De plus, pour éviter tout contact avec la virole de force, le gaz très chaud du démarrage de la réaction est introduit dans la chambre de catalyse, soit par l'intermédiaire d'une boîte résistant à la haute pression, mais de faible diamètre, et calorifugée intérieurement, soit à l'aide d'une tuyauterie interne. De même le gaz très chaud effluent de la chambre de catalyse est évacué soit par l'intermédiaire d'une boîte de mêmes caractéristiques que précédemment soit à l'aide d'une tuyauterie interne.

Dans ce type de réacteur chimique adiabatique, adapté aux réactions exothermiques, sous pression élevée, en phase gazeuse et catalyse hétérogène, les gaz réactifs circulent verticalement, de haut en bas, ou de bas en haut, à travers le lit catalytique. La forme du réacteur et la direction de circulation des gaz dans la zone de catalyse par rapport à l'axe de l'enceinte réactionnelle, sont adaptées au volume de catalyseur de façon à augmenter la section de passage des gaz à travers le catalyseur, diminuer donc fortement la perte de charge et ainsi permettre l'utilisation de catalyseur de granulométrie très petite.

Les réactifs et les effluents sont acheminés via deux tuyauteries internes, à travers des canaux de distribution assurant une bonne répartition du gaz dans la masse catalytique. A l'intérieur de l'enceinte porte-catalyseur, dans sa partie inférieure, sont disposés deux canaux-collecteurs de gaz reliés à sa tubulure des gaz réactionnels effluents.

La mise en place et le retrait de la grande quantité de catalyseur s'effectuent par l'orifice ménagé à la partie supérieure de la chambre de catalyse, de préférence au centre de celle-ci, correspondant à une tubulure ou virole interne soudée sur la virole principale de l'enceinte. Ces opérations d'introduction ou de récupération du catalyseur usagé sont facilitées par au moins deux cloisons amovibles verticales, constituées d'au moins deux secteurs de cercle emboîtables, qui ménagent temporairement entre elles un espace de travail. Ceci permet également d'araser à distance le catalyseur aux extrémités de l'enceinte.

En raison de ce mode de remplissage, ce type de réacteur horizontal est bien adapté aux couches catalytiques de grand et très grand volumes.

Le taux de remplissage de ces réacteurs horizontaux unicouches est très élevé, de l'ordre de 0,7 à 0,9.

Les opérations de manutention du catalyseur sont simplifiées et les engins de levage sont de portée et de puissance très modestes. En effet, l'équipement interne étant introduit dans le réacteur lors de la construction, il n'est jamais nécessaire, même au montage sur le site, d'introduire ou sortir le panier porte-catalyseur, voire même l'équipement interne complet. On parvient à supprimer les risques de fuite d'hydrogène par l'adoption d'un orifice de remplissage et de vidange du catalyseur de faible diamètre obturé par un couvercle soudé pouvant être découpé, ou par un couvercle à bride de petit diamètre à joint soudé. Le changement de catalyseur étant une opération de moins en moins fréquente, il peut être intéressant d'utiliser un couvercle soudé.

Les problèmes de dilatations longitudinales sont résolus en soudant verticalement la virole interne, d'introduction du catalyseur, sur la virole principale de l'enceinte catalytique, cette virole interne étant équipée de moyens, tels que des vis diamétralement opposées qui permettent par serrage-appui sur le corps de force, d'assurer un point fixe ; ce point n'étant pas fixe par rapport aux dilatations verticales qui peuvent s'effectuer librement.

Suivant les volumes de catalyseur, le taux de conversion, la perte de charge et d'autres critères technico-économiques spécifiques, il est avantageux de proposer une association de réacteurs catalytiques contenant chacun un seul lit de catalyseur avec circulation verticale des gaz réactifs, et dépourvus d'échangeur interne. Ces divers réacteurs associés en série ou parfois en parallèle à un ou plusieurs réacteurs horizontaux, peuvent être du type vertical ou sphérique. On peut envisager plusieurs réacteurs horizontaux en série, en général deux ou trois, ou pour des volumes de catalyseur couramment employés, un premier réacteur, soit vertical, soit sphérique et un deuxième réacteur horizontal qui peut être suivi d'un autre réacteur horizontal.

Dans une petite installation dont le coût d'investissement minimum, la simplicité du schéma seraient recherchés, sans récupération énergétique poussée, on peut utiliser deux réacteurs en série et, suivant les volumes de catalyseur, un premier réacteur, soit vertical, soit sphérique, et un deuxième réacteur vertical ou sphérique ou horizontal. Le refroidissement entre réacteurs pourra être direct, c'est-à-dire assuré par une trempe. En effet, la diminution du taux de conversion, due à l'emploi d'une trempe, et l'augmentation consécutive de la taille des équipements de la boucle de synthèse sont largement compensées par l'absence de l'échangeur intermédiaire en acier inoxydable. Le taux de conversion est maintenu à une valeur élevée par l'utilisation de granulométries très petites. Ceci est rendu possible par la forme des réacteurs, comme on l'a vu précédemment.

Au contraire, dans une grande installation où le coût énergétique serait déterminant, on pourra utiliser des réacteurs en série, de grande capacité, et éventuellement, en plus grand nombre (trois ou quatre pratiquement). Suivant les volumes de catalyseur, le ou les premiers réacteurs seront sphériques ou horizontaux, le ou les derniers réacteurs seront généralement horizontaux, les réacteurs non horizontaux pouvant être multicouches. Le taux de conversion sera augmenté par l'utilisation d'un refroidissement entre couches indirect, par échange thermique. La récupération thermique pourra être optimisée par l'utilisation d'une ou plusieurs chaudières haute pression sur un ou plusieurs des effluents des réacteurs en série, les autres effluents étant refroidis dans des échangeurs de chaleur par le gaz d'alimentation du premier lit. Cette combinaison de réacteurs et d'autres appareils permet alors un taux de conversion global plus élevé. La perte de charge globale est plus faible que dans les plus performants des réacteurs multi ou unicouches actuels.

Entre ces deux exemples extrêmes, toutes les combinaisons de types de réacteurs unicouches verticaux, horizontaux ou sphériques, de refroidissement direct par trempe ou indirect par échange de chaleur, de récupération thermique de la chaleur de réaction par une ou plusieurs chaudières ou réchauffeur d'eau haute pression et échangeurs de chaleur avec le gaz d'alimentation, sont possibles. Cela signifie que cette technique autorise une adéquation parfaite aux contraintes technico-économiques quelles qu'elles soient, avec un rendement supérieur.

Il est donné ci-après des exemples de réalisation non limitatifs de réacteurs unicouches horizontaux du type décrit dans l'invention, adaptés à la synthèse de l'ammoniac et du méthanol. Plus spécifiquement, dans la description ci-après les températures indiquées et les termes gaz de synthèse ou protection, gaz après réaction ou effluent réactionnel concernent la synthèse de l'ammoniac. Le type de réacteur est compatible avec toutes les pressions, et pratiquement il peut être avantageusement mis en œuvre entre 50 bars et 350 bars.

Exemple 1

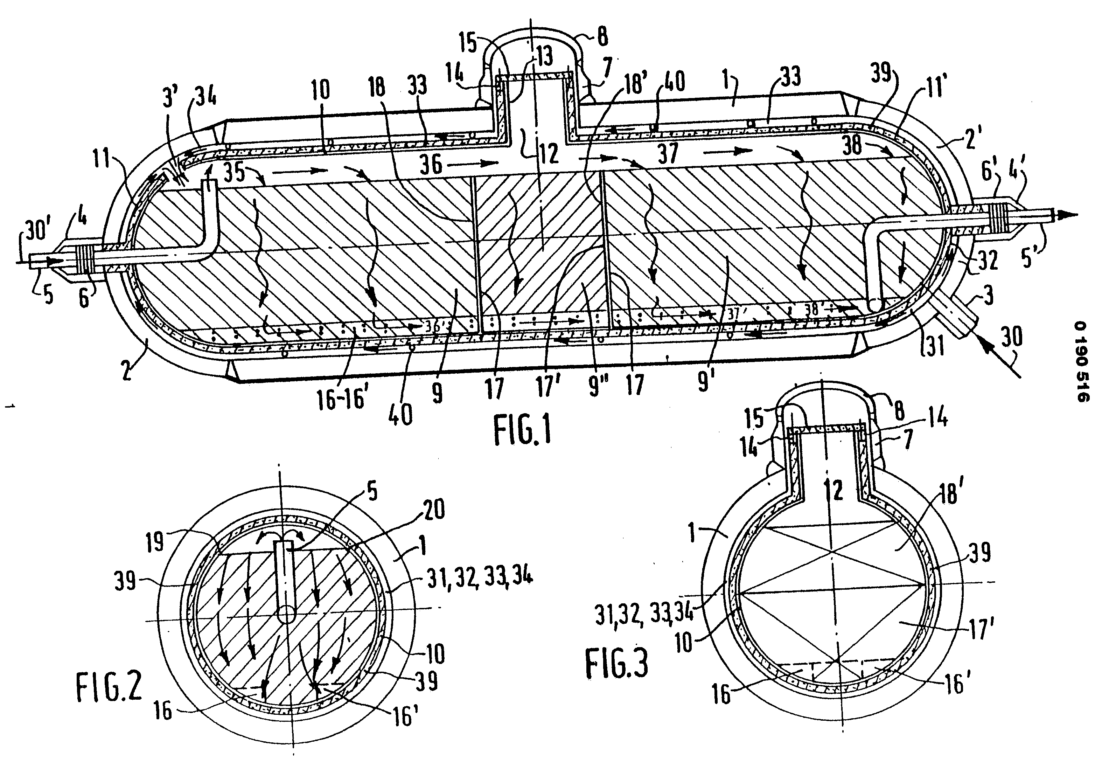

Réacteur unicouche horizontal à température de paroi contrôlée à un niveau de l'ordre de 400 °C, représenté sur les figures 1, 2 et 3 du dessin annexé.

Le corps du réacteur est constitué d'une virole en acier ou corps de force (1) et de deux fonds hémisphériques en acier du même type (2) et (2').

Le fond (2') est équipé d'une tubulure (3) d'entrée de gaz de synthèse ou de protection, préchauffé à 400 °C au maximum, et utilisé pour maintenir le corps de force à une température inférieure à celle de la chambre de catalyse, d'une tubulure (4') formant manchon thermique et boîtier pour les compensateurs (6') de la tubulure axiale horizontale de sortie de gaz chaud effluent de synthèse (5') à la température d'environ 500 °C, entre 450-530 °C.

Le fond (2) est équipé d'une tubulure (4) formant manchon thermique et boîtier pour les compensateurs (6) de la tubulure d'entrée (5) axiale horizontale, diamétralement opposé à la tubulure (5') et introduisant le mélange réactionnel préchauffé pour le démarrage de la réaction.

La virole (1), au cente de sa partie supérieure, est équipée d'une tubulure (7) de grand diamètre, adaptée au remplissage et à la vidange du catalyseur, un fond soudé (8) obture cette tubulure.

A l'intérieur du réacteur est disposée une capacité ou chambre de catalyse (10, 11, 11') concentrique au corps de force, destinée à recevoir le catalyseur (9, 9', 9"). La capacité non démontable est mise en place dans le corps du réacteur avant soudure du fond sphérique (2'). Cette capacité est constituée d'une partie cylindrique (10) ou virole et de deux fonds hémisphériques (11 et 11'), le tout calorifugé extérieurement. Un orifice (12) est prévu au centre à la partie supérieure. Cet orifice correspond à la tubulure (7) par laquelle est introduite la virole (13) terminée par une bride (14) et une bride pleine (15).

Cette tubulure (13) soudée sur la virole (10) est équipée de moyen, tel que des vis diamétralement opposées qui permettent par serrage-appui sur le corps de force (1) d'assurer un point fixe répartissant ainsi les dilatations longitudinales, tout en laissant à la dilatation dans le sens vertical la latitude de s'effectuer librement.

A l'intérieur de la capacité, dans sa partie inférieure sont disposés deux canaux-collecteurs de gaz (16 et 16') reliés à la tubulure des gaz réactionnels effluents (5').

Dans la partie centrale de la chambre réactionnelle (10) deux cloisons amovibles (17-18) et (17'-18') constituées de deux secteurs de cercles emboîtables sont disposées verticalement afin de faciliter la mise en place du catalyseur qui s'effectue par les orifices (7) et (12) en commençant par les extrémités, les cloisons amovibles étant déposées. Le catalyseur étant arasé au niveau des deux plats (19) et (20) figure 2, lorsque le remplissage, compte-tenu du talus d'éboulement arrive à l'emplacement des cloisons, les cloisons (17) et (17') sont montées, le remplissage est poursuivi, ensuite les cloisons (18) et (18') sont montées, la fin du remplissage et l'arasement en (9) et (9') sont réalisés, puis on effectue le remplissage en (9"), enfin l'orifice interne (12) est obturé par le couvercle (15) et l'orifice externe (7) par soudure du fond (8).

La protection thermique du corps de force (1) est assurée par un calorifuge (39) disposé à l'extérieur de la virole (10) et protégé par une série de viroles assemblées sur des frettes-support circulaires. Le type de calorifuge adapté possède, outre ses qualités isolantes une bonne résistance à la compression. Le calorifuge assume la fonction de supportage de l'enceinte porte-catalyseur, ce qui permet de supprimer tous les ponts thermiques entre la virole (10) contenant le catalyseur et la virole protégeant le calorifuge (39). En outre, le corps de force (1) est aussi protégé thermiquement par la circulation du gaz de protection (30), sous pression, entrant à 400 °C par la tubulure (3) puis pénétrant à l'intérieur de la chambre catalytique par la tubulure interne (3') diamétralement opposée. Ce gaz de protection traverse les trous ménagés dans les frettes-supports, et son circuit est canalisé, entre les spires d'une tige métallique (40) à enroulement hélicoïdal, dans l'espace (31, 32, 33, 34) ménagé entre la paroi interne de la virole (1) et des fonds hémisphériques (2) et (2') et la paroi externe protégeant le calorifuge (39), concentrique à la chambre catalytique.

Au démarrage de l'installation, le gaz (30) de protection thermique de la paroi du corps de force (1, 2, 2'), sous pression entre en (3) à 400 °C, circule en (31, 32, 33, 34) et pénètre par la communication (3') dans la chambre catalytique à une température pratiquement de l'ordre de 400 °C dans la zone libre (35) au-dessus du catalyseur.

Le gaz de synthèse (30'), préalablement réchauffé à l'extérieur jusqu'à une température maximale de 500 °C pour l'amorçage entre dans le réacteur par l'intermédiaire de la tubulure (5), se mélange avec le gaz de protection (30) dans l'espacement (35) entre la surface supérieure du catalyseur et la paroi interne de la chambre catalytique, le mélange réactionnel se répartit en (36, 37, 38) dans le canal distributeur de gaz réactifs, traverse les zones de catalyseur (9, 9', 9"). Après réaction, l'effluent de synthèse chaud (35', 36', 37', 38') est capté par les canaux (16) et (16') d'où il ressort par l'intermédiaire du collecteur-tubulure (5') à une température de l'ordre de 500 °C, suivant le régime de marche entre 490 et 520 °C.

En marche, après amorçage de la réaction, l'injection en (5) est arrêtée et seul le gaz de synthèse ayant joué le rôle de gaz de protection (30) continue à circuler à travers le passage (3') à température voisine de 400 °C, puis à entrer en (35) dans la chambre catalytique et passer sur le catalyseur puis ressortir en (5') à une température de 500 °C au maximum.

Exemple 2

Réacteur unicouche horizontal à température de paroi contrôlée à un niveau très modéré de l'ordre de 200 °C.

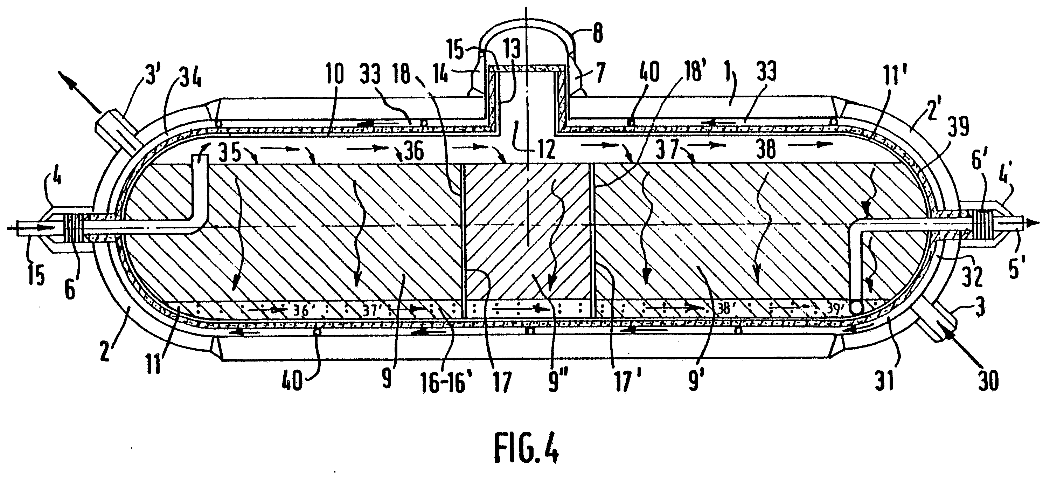

Ce réacteur représenté par les figures 2, 3 et 4 du dessin annexé, est une variante du réacteur précédemment décrit, quant à la structure de circulation du gaz de protection thermique et la température de celui-ci.

Le corps du réacteur est constitué d'une virole (1) de deux fonds hémisphériques (2) et (2'). Le fond (2') est équipé d'une tubulure (3) d'entrée du gaz frais, ou gaz de synthèse ou de protection à une température voisine de 200 °C, qui maintient le corps de force (1) à une température très modérée, d'une tubulure (4') formant manchon thermique et boîtier pour les compensateurs (6') de la tubulure axiale horizontale de sortie du gaz chaud (5') après réaction à 500 °C environ.

Le fond (2) est équipé d'une tubulure (3') de sortie du gaz de protection à température légèrement supérieure de quelques degrés à la température d'entrée diamétralement opposée à la tubulure (3), d'une tubulure (4) formant manchon thermique et boîtier pour les compensateurs (6) de la tubulure d'entrée (5) de gaz de synthèse avant réaction, diamétralement opposée à la tubulure de sortie (5') des gaz chauds après réaction.

Comme sur la réalisation précédente le corps de force (1, 2, 2') est équipé de la tubulure (7) et du fonds soudé (8). La chambre de catalyse (10, 11, 11') est disposée à l'intérieur dudit corps de force, celle-ci est munie des orifice (12), tubulure (7), virole (13), bride (14) et couvercle (15) ayant les mêmes fonctions que sur le réacteur de l'exemple 1. Les canaux (16 et 16') sont disposés de la même manière. Et, la chambre catalytique est munie des cloisons amovibles (emboîtables (17-18) (17'-18'), le remplissage du catalyseur (9, 9', 9") et son arasement en (19) et (20) s'effectuent suivant la technique déjà décrite.

La protection thermique du corps de force est assurée comme précédemment par le même type de calorifuge (39). Elle est aussi assurée par la circulation du gaz de protection (30) entrant à 200 °C en (3) et ressortant par la tubulure (3') à une température supérieure de quelques degrés. Ce circuit du gaz (30) est canalisé entre les spires de la tige métallique (40) dans l'espace (31, 32, 33, 34).

Au démarrage de l'installation, le gaz de protection, ayant la composition du gaz de synthèse entre en (3) à température de l'ordre de 200 °C, circule en (31, 32, 33, 34) et ressort en (3') à 200 °C. Ce gaz préalablement réchauffé à l'extérieur du réacteur jusqu'à une température maximale de 500 °C entre par la tubulure (5) dans le réacteur, est amené en (35) et réparti dans le canal distributeur (36, 37, 38), traverse les zones de catalyseur (9, 9', 9"). Après réaction le gaz chaud constituant l'effluent réactionnel est capté par les canaux (16, 16') d'où il ressort par l'intermédiaire du collecteur précédant la tubulure (5') à 500 °C maximum. En marche normale, après amorçage de la réaction, le même processus est suivi excepté que le gaz de synthèse entrant n'est plus préchauffé à 500 °C maximum, mais est introduit à une température de l'ordre de 400 °C dans la chambre catalytique et ressort à une température d'environ 500 °C.

**Revendications**

1. Réacteur de synthèse catalytique unicouche exothermique en phase gazeuse sous pression, cylindrique, horizontal, caractérisé en ce qu'il est constitué par une virole principale sous haute pression ou corps de force, composée d'une partie cylindrique (1) et deux fonds hémisphériques (2) (2'), contenant une enceinte porte-catalyseur (10, 11, 11'), renfermant une seule couche de catalyseur (9, 9', 9"), concentrique au corps de force et dont la paroi externe est revêtue d'un isolant (39), un espace (31, 32, 33, 34) étant ménagé entre la paroi interne du corps de force et celle de la paroi externe de l'enceinte porte-catalyseur revêtue d'isolant, reposant sur des frettes et sur un enroulement métallique hélicoïdal (40) d'une extrémité à l'autre de la virole, la virole principale sous haute pression et l'enceinte porte-catalyseur étant munies de moyen d'introduction (3) et (5), et d'évacuation (3') et (5') des flux gazeux situés aux extrémités du grand axe et disposés en vis-à-vis sur les fonds hémisphériques, en outre l'enceinte porte-catalyseur étant équipée d'une tubulure verticale (13) de remplissage et vidange du catalyseur.

2. Réacteur de synthèse selon la revendication 1, caractérisé en ce que le revêtement extérieur isolant (39) de l'enceinte porte-catalyseur (10, 11, 11') hautement résistant à la compression dans les conditions de service assure le supportage de l'enceinte, ledit revêtement isolant (39) étant enfermé dans des viroles de protection mécanique, concentriques à l'enceinte porte-catalyseur (10, 11, 11'), lesdites viroles s'appuyant sur des frettes-supports dans lesquelles sont ménagés des orifices pour le passage des gaz.

3. Réacteur de synthèse selon la revendication 1, caractérisé en ce qu'il comporte deux tuyauteries internes (5) et (5') d'acheminement des gaz réactifs et effluents, et à l'intérieur de l'enceinte porte-catalyseur (10) dans sa partie supérieure un canal distributeur de gaz réactifs (35, 36, 37, 38) et dans sa partie inférieure des canaux-collecteurs (16) et (16') des gaz reliés à la tubulure (5') des gaz effluents.

4. Réacteur de synthèse selon la revendication 1, caractérisé en ce que l'enceinte porte-catalyseur (10) comporte au moins deux cloisons verticales amovibles (17, 18) et (17', 18') constituées d'au moins deux secteurs de cercle emboîtables, ménageant temporairement entre elles un espace de travail lors du remplissage du catalyseur.

5. Réacteur de synthèse selon la revendication 4, caractérisé en ce que la mise en place et le retrait du catalyseur s'effectuent par l'orifice (12) ménagé à la partie supérieure de la chambre de catalyse (10) (11) (11'), celui-ci correspondant à une tubulure ou virole (13), soudée sur la virole principale (10), la virole (13) étant elle-même équipée de moyens (14) permettant par serrage-appui sur la tubulure (7) du corps de force (1) d'assurer un point fixe par rapport aux dilatations longitudinales et l'orifice (12) étant obturé par un couvercle (15), la virole (7) étant obturée par un couvercle (8) soudé.

6. Réacteur de synthèse selon la revendication 1, caractérisé en ce qu'il est associé à au moins un réacteur du même type ou à au moins un réacteur vertical unicouche ou à tout autre type de réacteur.

7. Application du réacteur de synthèse selon la revendication 1 à la synthèse d'ammoniac et du méthanol.

8. Procédé de synthèse catalytique exothermique en phase gazeuse sous pression mis en œuvre dans le réacteur, selon l'une des revendications 1 à 5, selon lequel la réaction est conduite en présence d'une seule couche de catalyseur avec circulation verticale des gaz réactifs dans ladite couche, caractérisé en ce qu'un courant de gaz isotherme de température moins élevée que celle du gaz contenu dans l'enceinte interne porte-catalyseur, constitué par le gaz d'alimentation sous pression avant son introduction dans la chambre catalytique circule à l'extérieur de celle-ci, d'une extrémité à l'autre, en assurant la protection thermique de la paroi sous haute pression.

9. Procédé de synthèse catalytique selon la revendication 8, caractérisé en ce que le gaz de protection thermique, sous pression, circule de façon hélicoïdale.

10. Procédé de synthèse catalytique d'ammoniac, selon la revendication 8, caractérisé en ce que le gaz de protection thermique, sous pression, en fin de circuit est introduit directement à la synthèse à une température comprise entre 370 et 420 °C.

11. Procédé de synthèse catalytique d'ammoniac, selon la revendication 8, caractérisé en ce que le gaz de protection thermique sous pression, circulant à une température comprise entre 120 et 250 °C, en fin de circuit est réchauffé extérieurement avant d'être réintroduit dans l'enceinte catalytique à un niveau de température d'entrée en réaction.

12. Procédé de synthèse catalytique d'ammoniac, selon la revendication 10, caractérisé en ce que dans la phase d'amorçage de la réaction, on introduit dans la chambre catalytique un gaz d'alimentation sous pression, à une température au plus égale à 500 °C, en cours de marche la température d'entrée du gaz réactif dans la chambre catalytique est de l'ordre de 400 °C.

13. Procédé de synthèse catalytique d'ammoniac, selon la revendication 11, caractérisé en ce que dans la phase de démarrage, le gaz réactif est constitué par le mélange du gaz de synthèse sous pression, préalablement réchauffé à l'extérieur et du gaz de protection isothermique sous la même pression.

14. Procédé de synthèse catalytique d'ammoniac, selon la revendication 13, caractérisé en ce que dans la phase de marche normale, le gaz réactif est uniquement constitué par le gaz de protection thermique.

**Claims**

1. Cylindrical and horizontal reactor for pressurised exothermal single-layer gas-phase catalytic synthesis, characterised in that it is formed by a main casing under high pressure or a force-resistant member, composed of a cylindrical part (1) and of two hemispherical end members (2) (2'), containing a catalyst-supporting chamber (10, 11, 11') carrying a single layer or bed of catalyst (9, 9', 9"), concentric with the force-resistant member and of which the external wall is lined with an insulating material (39), a space (31, 32, 33, 34) being formed between the internal wall of the force-resistant member and that of the external wall of the insulator-lined catalyst-supporting chamber, resting on hoops and on a helical metallic winding (40) from one end to the other of the casing, the main casing under high pressure and the catalyst-supporting chamber being provided with introduction means (3) and (5) and discharge means (3') and (5') for the gaseous flows, situated at the ends of the main axis and disposed facing one another on the hemispherical end members, the catalyst-carrying chamber also being equipped with a vertical socket (13) for the charging with and emptying of the catalyst.

2. Synthesis reactor according to claim 1, characterised in that the insulating external lining (39) of the catalyst-supporting chamber (10, 11, 11'), highly resistant to compression under the operating conditions, assures the supporting of the chamber, the said insulating lining (39) being confined in mechanical protection casings concentric with the catalyst-supporting chamber (10, 11, 11'), the said casings bearing on support hoops in which are formed orifices for the passage of gases.

3. Synthesis reactor according to claim 1, characterised in that it comprises two internal pipes (5) and (5') for the passage of reactive gases and effluents, and within the catalyst-supporting chamber (10), in its upper part, a distributor duct (35, 36, 37, 38) for reactive gases and, in its lower part, gas-collector channels (16) and (16') connected to the effluent gas nozzle (5').

4. Synthesis gas reactor according to claim 1, characterised in that the catalyst-supporting chamber (10) comprises at least two removable vertical partitions (17, 18) and (17', 18') formed of at least two sectors of a circle which can be interfitted and which temporarily form between them a working space at the time of refilling with catalyst.

5. Synthesis reactor according to claim 4, characterised in that the positioning and the withdrawal of the catalyst are effected by way of the orifice (12) formed at the upper part of the catalysis chamber (10) (11) (11'), said orifice corresponding to a tube or nozzle (13) welded on the main chamber (10), the tube (13) being itself equipped with means (14) which, by locking engagement on the nozzle (7) of the member (1), makes it possible to ensure a fixed point with respect to the longitudinal expansions, the opening (12) being closed by a cover (15) and the nozzle (7) being closed by a welded cover (8).

6. Synthesis reactor according to claim 1, characterised in that it is associated with at least one reactor of like type or with at least one single-layer vertical reactor or any other type of reactor.

7. Application of the synthesis reactor according to claim 1 to the synthesis of ammonia and methanol.

8. Process for pressurised exothermal gas-phase catalytic synthesis carried out in the reactor according to one of the claims 1 to 5, according to which the reaction is conducted in the presence of a single layer of catalyst with vertical circulation of the reactive gases in the said layer, characterised in that a flow of isothermal gas at a temperature lower than that of the gas contained in the catalyst-carrying internal chamber, formed by the supply gas under pressure before its introduction into the catalytic chamber, circulates externally of this latter, from one end to the other, thereby assuring the thermal protection of the wall under high pressure.

9. Catalytic synthesis process according to claim 8, characterised in that the thermal protection gas, under pressure, circulates in helical form.

10. Process for catalytic synthesis of ammonia, according to claim 8, characterised in that the thermal protection gas under pressure, on completing the circuit, is introduced directly to the synthesis at a temperature which is between 370 and 420 °C.

11. Process for catalytic synthesis of ammonia, according to claim 8, characterised in that the thermal protection gas under pressure, circulating at a temperature which is between 120 and 250 °C, on completing the circuit, is reheated externally before being reintroduced into the catalytic chamber at a temperature level of entry into reaction.

12. Process for catalytic synthesis of ammonia, according to claim 10, characterised in that, in the initiation phase of the reaction, a supply gas under pressure is introduced into the catalytic chamber, at a temperature at most equel to 500 °C, and during operation, the entry temperature of the reactive gas into the catalytic chamber is of the order of 400 °C.

13. Process for catalytic synthesis of ammonia, according to claim 11, characterised in that, in the starting phase, the reactive gas is formed by the mixture of the sub-pressure synthesis gas, previously re-heated externally, and the isothermic protection gas under the same pressure.

14. Process for catalytic synthesis of ammonia, according to claim 13, characterised in that, in the normal operating phase, the reactive gas is formed solely by the thermal protection gas.

**Patentansprüche**

1. Zylindrischer horizontaler Reaktor für katalytische einschichtige exotherme Synthese in der Gasphase unter Druck, dadurch gekennzeichnet, daß er aus einem Hauptmantel unter Hochdruck oder Druckkörper aus einem zylindrischen Teil (1) und zwei halbkugelförmigen Böden (2) (2') besteht, der einen Katalysatorraum (10, 11, 11') enthält, welcher eine einzige Katalysatorschicht (9, 9', 9") einschließt, konzentrisch zu dem Druckkörper ist und dessen Außenwand von einem Isoliermaterial (39) umhüllt ist, wobei ein Raum (31, 32, 33, 34) zwischen der Innenwand des Druckkörpers und der Außenwand des mit Isoliermaterial umhüllten Katalysatorraumes angeordnet ist und auf Ringarmierungen und auf einer metallischen spiralförmigen Wicklung (40) von einem Ende des Mantels zum anderen ruht, der Hauptmantel unter Hochdruck und der Katalysatorraum mit Mitteln zur Einführung (3) und (5) und zur Entfernung (3') und (5') von Gasströmen an den Enden der großen Achse und gegenüber den halbkugelförmigen Böden gelegen sind und außerdem der Katalysatorraum mit einem vertikalen Stutzen (13) zum Einfüllen und Entleeren von Katalysator ausgestattet ist.

2. Synthesereaktor nach Anspruch 1, dadurch gekennzeichnet, daß die äußere isolierende Umhüllung (39) des Katalysatorraumes (10, 11, 11'), die sehr beständig gegen die Kompression bei den Betriebsbedingungen ist, die Unterstützung des Raumes gewährleistet, wobei die isolierende Umhüllung (39) in zum Katalysatorraum (10, 11, 11') konzentrischen mechanischen Schutzmänteln eingeschlossen ist und diese Mäntel auch auf Armierungsauflagen ruhen, in welchen Öffnungen für den Gasdurchgang vorgesehen sind.

3. Synthesereaktor nach Anspruch 1, dadurch gekennzeichnet, daß er zwei innere Rohrleitungen (5) und (5') zur Beförderung von Reaktionsgasen und Ausläufen sowie im Inneren des Katalysatorraumes (10) in dessen oberem Teil einem Verteilerkanal für Reaktionsgas (35, 36, 37, 38) und in seinem unteren Teil Sammelkanäle (16) und (16') für Gas, die mit dem Gasauslaufstutzen (5') in Verbindung stehen, aufweist.

4. Synthesereaktor nach Anspruch 1, dadurch gekennzeichnet, daß der Katalysatorraum (10) wenigstens zwei vertikale lösbare Zwischenwände (17, 18) und (17', 18') aufweist, die aus wenigstens zwei einpaßbaren Kreissektoren bestehen, wobei zwischen ihnen vorübergehend während des Katalysatoreinfüllens ein Arbeitsraum angeordnet ist.

5. Synthesereaktor nach Anspruch 4, dadurch gekennzeichnet, daß die Einführung und die Entnahme von Katalysator durch die Öffnung (12) erfolgen, die im oberen Teil der Katalysatorkammer (10), (11), (11') vorgesehen ist, wobei diese einem Stutzen oder Ring (13) entspricht, der auf den Hauptmantel (10) aufgeschweißt ist, wobei der Ring (13) selbst mit Einrichtungen (14) ausgestattet ist, die durch Klemmhalterung auf dem Stutzen (7) des Druckkörpers (1) es gestatten, einen festen Punkt gegenüber Längsausdehnungen zu bilden, und wobei die Öffnung (12) durch einen Deckel (15) verschlossen und der Ring (7) durch einen aufgeschweißten Deckel (8) verschlossen ist.

6. Synthesereaktor nach Anspruch 1, dadurch gekennzeichnet, daß er mit wenigstens einem Reaktor gleichen Typs oder mit wenigstens einem vertikalen Einschichtreaktor oder mit irgendeinem anderen Reaktortyp verbunden ist.

7. Verwendung des Synthesereaktors nach Anspruch 1 für die Synthese von Ammoniak und von Methanol.

8. Verfahren zur katalytischen exothermen Synthese in Gasphase unter Druck in dem Reaktor nach einem der Ansprüche 1 bis 5, bei dem die Umsetzung in Gegenwart einer einzigen Katalysatorschicht unter vertikaler Zirkulation der Reaktionsgase in dieser Schicht durchgeführt wird, dadurch gekennzeichnet, daß ein isothermer Gasstrom von weniger hoher Temperatur als die des in dem inneren Katalysatorraum enthaltenen Gases, der aus dem Einspeisgas unter Druck vor seiner Einführung in die katalytische Kammer besteht, außerhalb derselben von einem Ende zum anderen zirkuliert und so den Wärmeschutz der Wand unter Hochdruck gewährleistet.

9. Katalytisches Syntheseverfahren nach Anspruch 8, dadurch gekennzeichnet, daß das Wärmeschutzgas unter Druck in spiralförmiger Weise zirkuliert.

10. Verfahren zur katalytischen Ammoniaksynthese nach Anspruch 8, dadurch gekennzeichnet, daß das Wärmeschutzgas unter Druck am Ende des Kreislaufs direkt in die Synthese mit einer Temperatur zwischen 370 und 420 °C eingeführt wird.

11. Verfahren zur katalytischen Synthese von Ammoniak nach Anspruch 8, dadurch gekennezeichnet, daß das Wärmeschutzgas unter Druck, das bei einer Temperatur zwischen 120 und 250 °C zirkuliert, am Ende des Kreislaufes außerhalb wiedererwärmt wird, bevor es wieder in den Katalysatorraum mit einem Temperaturniveau für den Reaktionseintritt eingeführt wird.

12. Verfahren zur katalytischen Synthese von Ammoniak nach Anspruch 10, dadurch gekennzeichnet, daß man in der Phase der Einleitung der Reaktion in die Katalysatorkammer Einspeisgas unter Druck bei einer Temperatur von höchstens 500 °C einführt und daß im Verlauf des Betriebs die Eintrittstemperatur des Reaktionsgases in die Katalysatorkammer in der Größenordnung von 400 °C ist.

13. Verfahren zur katalytischen Synthese von Ammoniak nach Anspruch 11, dadurch gekennzeichnet, daß in der Einleitungsphase das Reaktionsgas aus dem Gemisch von Synthesegas unter Druck, das vorher außerhalb wiedererwärmt wurde, und isothermen Schutzgas unter dem gleichen Druck besteht.

14. Verfahren zur katalytischen Synthese von Ammoniak nach Anspruch 13, dadurch gekennzeichnet, daß in der Phase des normalen Betriebs das Reaktionsgas alleine aus dem Wärmeschutzgas besteht.

FIG.1

FIG.2

FIG.3

0 190 516

0 190 516

FIG.4